# EUROPÄISCHE PATENTSCHRIFT

(11) **EP 3 358 483 B1**
(45) Veröffentlichungstag und Bekanntmachung des Hinweises auf die Patenterteilung: **20.10.2021**
(21) Anmeldenummer: 17154830.8
(22) Anmeldetag: 06.02.2017
(51) Int. Cl.: G16H 40/67

(54) **ÜBERTRAGEN EINES DATENSATZES**
TRANSFER OF A DATA SET
TRANSMISSION D'UN ENSEMBLE DE DONNÉES

(43) Veröffentlichungstag der Anmeldung: 08.08.2018
(73) Patentinhaber: Siemens Healthcare GmbH, 91052 Erlangen (DE)
(72) Erfinder: Hegendörfer, Friedrich, 91365 Weilersbach (DE); Korinth, Martin, 85604 Zorneding (DE)

(56) Entgegenhaltungen:
- DE-A1-102013 003 044
- US-A1- 2007 181 691
- US-A1- 2012 217 293
- ISHIHARA TAKAYUKI ET AL: "Compatible 2D-Code Having Tamper Detection System with QR-Code", 2014 TENTH INTERNATIONAL CONFERENCE ON INTELLIGENT INFORMATION HIDING AND MULTIMEDIA SIGNAL PROCESSING, IEEE, 27. August 2014 (2014-08-27), Seiten 493-496, XP032715264, DOI: 10.1109/IIH-MSP.2014.129 [gefunden am 2014-12-24]
- KUAN-CHIEH LIAO ET AL: "A One-Time Password Scheme with QR-Code Based on Mobile Phone", INC, IMS AND IDC, 2009 FIFTH INTERNATIONAL JOINT CONFERENCE ON, IEEE, PISCATAWAY, NJ, USA, 25. August 2009 (2009-08-25), Seiten 2069-2071, XP031564781, ISBN: 978-0-7695-3769-6

## Beschreibung

In vielen Bereichen der Technik ist es notwendig, Informationen über eine beim Kunden installierte Vorrichtung zentral beim Hersteller der Vorrichtung oder bei einem Dienstleistungsanbieter in einer Datenbank zu speichern. Solche Datenbanken können einerseits notwendig sein, um regulatorischen Anforderungen zu genügen, andererseits können sie aber auch zur Verbesserung von erbrachten Dienstleistungen verwendet werden. Von besonderer Bedeutung ist hierbei, die derzeitige Konfiguration der Vorrichtung, umfassend die verbauten körperlichen Komponenten (die sog. "Hardware") und die verwendete Steueranweisungen (die sog. "Software"), oder den aktuellen Zustand oder Status der Vorrichtung zu speichern. Eine solche Konfiguration kann sich aber in der Gebrauchszeit der Vorrichtung ändern. Um stets die aktuelle Konfiguration der Vorrichtung zu speichern, muss die Vorrichtung daher überwacht werden.

Bei derartigen zu überwachenden Vorrichtungen handelt es sich um medizintechnische Anlagen, beispielsweise um Ultraschallgeräte oder um fahrbare Röntgengeräte, für die regelmäßig besonders strenge regulatorische Anforderungen gelten.

Insbesondere aus Kostengründen ist es möglich, dass auch der Benutzer einer Vorrichtung selbst auf Anweisung beispielsweise des Herstellers Änderungen an der Konfiguration der Vorrichtung durch Hard- und/oder Softwareupdates durchführt. Der Erfolg oder Misserfolg dieser Konfigurationsänderungen müssen dann dem Hersteller der Vorrichtung oder einem Dienstleistungsanbieter zur Kenntnis gebracht werden. Auch müssen Informationen über Änderung von Zuständen (z.B. das Auftreten eines Fehlerzustands) an den Hersteller der Vorrichtung oder den Dienstleistungsanbieter gesendet werden.

Hierzu ist es bekannt, die überwachte Vorrichtung mit dem Internet zu verbinden, sodass die überwachte Vorrichtung eine Rückmeldung über den Erfolg oder den Misserfolg der Konfigurationsänderung geben kann. Wegen Sicherheitsbedenken, regulatorischen Anforderungen oder aus Kostengründen gibt es aber Vorrichtungen, die nicht mit dem Internet verbunden sind, beispielsweise mobile Ultraschallgeräte. Bei solchen, insbesondere bei mobilen Vorrichtungen, ist eine Rückmeldung über das Internet daher nicht möglich.

Weiterhin ist es bekannt, dass Anwender der Vorrichtung eine Rückmeldung über die erfolgte Konfigurationsänderung an den Hersteller der Vorrichtung oder den Dienstleistungsanbieter übermitteln, beispielsweise über Telefon, Fax oder E-Mail, und dabei die Daten der Konfigurationsänderung manuell und in einem vorgegebenen oder frei gewähltem Format erfassen. Eine solche Rückmeldung ist aber zeitintensiv und damit mit Kosten verbunden. Weiterhin können durch Übertragungsfehler oder durch absichtliche Manipulation des Anwenders falsche Informationen beim Hersteller der Vorrichtung oder beim Dienstleistungsanbieter gespeichert werden.

Aus der Druckschrift DE 10 2013 003 044 A1 ist bekannt, durch die Darstellung von optischen Codeinformationen und deren Übermittlung durch ein mobiles Endgerät Fahrzeugdiagnoseinformation eines Fahrzeuges an ein Servicecenter zu übertragen.

Es ist daher die Aufgabe der vorliegenden Erfindung, eine schnelle, kostengünstige und manipulationssichere Methode bereitzustellen, den Erfolg einer Konfigurationsänderung an eine zentrale Datenerfassungseinheit zu übertragen.

Die Aufgabe wird gelöst durch ein Verfahren nach Anspruch 1, ein Datenübertragungssystem nach Anspruch 13, ein Computerprogrammprodukt nach Anspruch 15 sowie durch ein computerlesbares Speichermedium nach Anspruch 16.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung basiert darauf, dass eine Konfigurationsänderung an einer überwachten Vorrichtung mittels einer ersten Recheneinheit einer überwachten Vorrichtung festgestellt wird. Weiterhin wird ein erster Datensatz mittels der ersten Recheneinheit der überwachten Vorrichtung bestimmt, wobei der erste Datensatz wenigstens eine öffentliche Geräteinformation und eine Konfigurationsinformation umfasst, und wobei die öffentliche Geräteinformation der überwachten Vorrichtung eineindeutig zuordenbar ist. Weiterhin wird eine visuelle Information basierend auf dem ersten Datensatz mittels der ersten Recheneinheit der überwachten Vorrichtung bestimmt. Weiterhin wird die visuelle Information von einer Ausgabeeinheit der überwachten Vorrichtung zu einer Eingabeeinheit einer mobilen Überwachungseinheit optisch übertragen. Weiterhin wird ein zweiter Datensatz aus der visuellen Information mittels einer zweiten Recheneinheit der mobilen Überwachungseinheit bestimmt, wobei aus dem zweiten Datensatz wenigstens die öffentliche Geräteinformation und die Konfigurationsinformation bestimmbar sind. Weiterhin wird der zweite Datensatz von einer Schnittstelle der mobilen Überwachungseinheit zu einer zentralen Datenerfassungseinheit übertragen. Hierbei kann die überwachte Vorrichtung insbesondere eine medizinische Vorrichtung sein, insbesondere ein bildgebendes Gerät, insbesondere ein mobiles Ultraschallgerät oder ein mobiles C-Bogen-Röntgengerät.

Die Erfinder haben erkannt, dass durch die Übertragung der öffentlichen Geräteinformation und der Konfigurationsinformation zu einer mobilen Überwachungseinheit eine Verbindung der mobilen Überwachungseinheit zur zentralen Datenerfassungseinheit, beispielsweise über das Internet, verwendet werden kann. Daher muss die überwachte Vorrichtung nicht direkt mit der zentralen Datenerfassungseinheit verbunden sein, dies führt zu einer Kostenersparnis und zu einer besseren Sicherheit der überwachten Vorrichtung. Weiterhin ist es durch die Verwendung einer optischen Übertragung nicht notwendig, eine dauerhafte Verbindung, beispielsweise eine kabelgebundene Verbindung oder eine Funkverbindung, zwischen der überwachten Vorrichtung und der mobilen Überwachungseinheit herzustellen. Die mobile Überwachungseinheit kann daher zum einen insbesondere zum Überwachen von mehreren überwachten Vorrichtungen verwendet werden, dies stellt eine Kostenersparnis dar. Durch die Verwendung der optischen Übertragung kann weiterhin die überwachte Vorrichtung nicht durch einen Dritten manipuliert werden, da keine Informationen und insbesondere kein Schadcode an die überwachte Vorrichtung übertragen werden kann. Durch das optische Übertragen ist es weiterhin nicht notwendig, die öffentliche Geräteinformation und die Konfigurationsinformation manuell zu erfassen oder weiterzugeben, sodass der Datensatz besonders schnell und effizient zu der zentralen Datenerfassungseinheit übertragen werden kann, zudem werden Flüchtigkeitsfehler des Anwenders beim Erfassen der Daten vermieden.

Nach einem weiteren Aspekt der Erfindung umfasst die Konfigurationsinformation wenigstens eine Information über den Erfolg der Konfigurationsänderung an der überwachten Vorrichtung. Der Erfolg einer Konfigurationsänderung ist insbesondere gegeben, wenn die Konfigurationsänderung erfolgreich durchgeführt wurde. Insbesondere ist es möglich, die visuelle Information nur darzustellen, wenn die Konfigurationsänderung der überwachten Vorrichtung erfolgreich war. Die Erfinder haben erkannt, dass mit einer derartigen Konfigurationsinformation besonders einfach ein korrekter Datenbestand in der zentralen Datenerfassungseinheit erreicht werden kann.

Nach einem weiteren Aspekt der Erfindung ist die visuelle Information in einem Bild enthalten, wobei beim optischen ersten Übertragen das Bild auf der Ausgabeeinheit der überwachten Anlage dargestellt wird und das Bild von der Eingabeeinheit der mobilen Überwachungseinheit erfasst wird. Eine visuelle Information ist insbesondere in einem Bild enthalten, wenn sie aus dem Bild alleine wiederhergestellt oder rekonstruiert werden kann. Eine visuelle Information ist insbesondere auch dann in einem Bild enthalten, wenn die visuelle Information in einer verschlüsselten oder codierten Form im Bild enthalten ist. Die Erfinder haben erkannt, dass das Ausgeben eines Bildes auf der Ausgabeeinheit der überwachten Vorrichtung sowie das Erfassen des Bildes mit der Eingabeeinheit der mobilen Überwachungseinheit besonders kostengünstig und effizient ist, da die geeignete Ausgabeeinheit und die geeignete Eingabeeinheit im Regelfall bereits vorhanden und für das Anzeigen oder Erfassen eines Bildes ausgebildet sind, und daher das Verfahren ohne kostenintensive Modifikation der überwachten Vorrichtung oder der mobilen Überwachungseinheit durchgeführt werden kann.

Nach einem weiteren Aspekt der Erfindung umfasst das Bild Bildbereiche, wobei ein Bildbereich entweder eine erste Farbe oder eine zweite Farbe aufweist, und wobei sich die erste Farbe und die zweite Farbe zumindest in ihrer Helligkeit und/oder in ihrem Farbton unterscheiden. Die erste Farbe und die zweite Farbe können sich auch insbesondere in ihrer Helligkeit unterscheiden. Die Erfinder haben erkannt, dass durch derartige Bildbereiche eine visuelle Information besonders effizient übertragen werden kann.

Nach einem weiteren Aspekt der Erfindung ist die visuelle Information in der geometrischen Anordnung der Bildbereiche des Bildes enthalten. Die Erfinder haben erkannt, dass diese geometrische Anordnung besonders gut, schnell und mit besonders wenigen Fehlern von der Eingabeeinheit der mobilen Überwachungseinheit erkannt werden kann.

Nach einem weiteren Aspekt der Erfindung umfasst das Bild einen eindimensionalen Barcode oder einen zweidimensionalen Barcode. Bei einem eindimensionalen Barcode kann es sich insbesondere um einen Strichcode handeln, bei einem zweidimensionalen Barcode kann es sich insbesondere um einen gestapelten Strichcode, einen Matrixcode oder eine Punktcode handeln. Bei einem zweidimensionalen Barcode kann es sich insbesondere um einen "Quick Response Code" (kurz "QR-Code", ein deutscher Begriff ist Schnellantwortcode) handeln. Ein eindimensionaler Barcode oder ein zweidimensionaler Barcode können insbesondere redundante Bereiche umfassen. Die Erfinder haben erkannt, dass sich durch einen eindimensionalen Barcode oder einen zweidimensionalen Barcode besonders effizient Informationen übertragen lassen. Weiterhin umfassen eindimensionale Barcodes und zweidimensionale Barcodes redundante Bereiche, sodass Fehler bei der Übertragung verhindert werden. Gleichzeitig wird durch die redundanten Bereiche auch die optische Übertragung beschleunigt, da Ungenauigkeiten in der Positionierung und der Bilderfassung durch die mobile Überwachungseinheit durch die Fehlerredundanz ausgeglichen werden können.

Nach einem weiteren Aspekt der Erfindung umfasst die öffentliche Geräteinformation eine erste Identifikationsnummer der überwachten Vorrichtung. Bei einer ersten Identifikationsnummer kann es sich insbesondere um eine Seriennummer der überwachten Vorrichtung handeln. Eine Identifikationsnummer kann insbesondere auch Zeichen umfassen, die nicht numerisch sind, beispielsweise Buchstaben oder Sonderzeichen. Die Erfinder haben erkannt, dass durch die Verwendung einer ersten Identifikationsnummer die an die zentrale Datenerfassungseinheit übertragenen Daten besonders einfach und damit schnell der überwachten Vorrichtung zugeordnet werden können.

Nach einem weiteren Aspekt der Erfindung umfasst die Konfigurationsänderung das Installieren und/oder das Entfernen einer Hardware- und/oder Softwarekomponente, wobei die Konfigurationsinformation eine zweite Identifikationsnummer der Hardware- und/oder Softwarekomponente umfasst. Die Erfinder haben erkannt, dass durch eine derartige Konfigurationsinformation besonders schnell und effizient die geänderte Konfiguration der überwachten Vorrichtung beschrieben werden kann. Insbesondere kann beim Vorhandensein mehrerer gleichwertiger Hard- und/oder Softwarekomponenten festgestellt werden, welche dieser Komponenten in welcher der überwachten Vorrichtungen verwendet wird.

Nach einem weiteren Aspekt der Erfindung ist die visuelle Information derart ausgebildet, dass eine Veränderung der Konfigurationsinformation und der öffentlichen Geräteinformation mittels der mobilen Überwachungseinheit und/oder mittels der zentralen Datenerfassungseinheit feststellbar ist. Die Erfinder haben erkannt, dass derart Übertragungsfehler oder absichtliche Manipulationen der Daten ausgeschlossen sind. Hierdurch kann die Konfigurationsinformation insbesondere fehlerfrei in der zentralen Datenerfassungseinheit gespeichert werden.

Nach einem weiteren Aspekt der Erfindung umfasst die visuelle Information wenigstens das Ergebnis der Anwendung einer Einwegstreuwertfunktion auf den ersten Datensatz. Ein englischer Fachbegriff für Einwegstreuwertfunktion ist "Hashfunktion". Die Erfinder haben erkannt, dass durch die Verwendung einer Einwegstreuwertfunktion besonders schnell die Veränderung der Konfigurationsinformation und der öffentlichen Geräteinformation festgestellt werden kann, weiterhin ist das Ergebnis der Anwendung einer üblichen Einwegstreufunktion kurz und daher schnell und kostengünstig zu übertragen.

Nach einem weiteren Aspekt der Erfindung umfasst der erste Datensatz weiterhin eine geheime Geräteinformation, wobei die geheime Geräteinformation der überwachten Vorrichtung eineindeutig zuordenbar ist, und wobei die geheime Geräteinformation nicht aus der visuellen Information bestimmbar ist. Die Erfinder haben erkannt, dass durch die Verwendung einer geheimen Geräteinformation eine Manipulation der übertragenen Daten ausgeschlossen werden kann.

Nach einem weiteren möglichen Aspekt der Erfindung ist die geheime Geräteinformation sowohl in der zentralen Datenerfassungseinheit als auch in der überwachten Vorrichtung gespeichert. Die Erfinder haben erkannt, dass durch eine derartige Speicherung eine Manipulation besonders einfach und kostengünstig ausgeschlossen werden kann.

Nach einem weiteren möglichen Aspekt der Erfindung umfasst die Konfigurationsänderung das Installieren und/oder das Entfernen einer Hardware- und/oder Softwarekomponente, weiterhin ist die geheime Geräteinformation sowohl in der zentralen Datenerfassungseinheit als auch in der Hardware- und/oder Softwarekomponente gespeichert. Die Erfinder haben erkannt, dass durch eine derartige Speicherung die Manipulation besonders gut ausgeschlossen werden kann, da für unterschiedliche Konfigurationsänderungen jeweils unterschiedliche geheime Geräteinformationen gewählt werden können.

Nach einem weiteren Aspekt der Erfindung ist die zweite Identifikationsnummer der überwachten Vorrichtung eineindeutig zuordenbar, wobei die zweite Identifikationsnummer sowohl in der zentralen Datenerfassungseinheit als auch in der neuen Hardware- und/oder Softwarekomponente gespeichert ist, und wobei die zweite Identifikationsnummer nicht aus der visuellen Information bestimmbar ist. Die Erfinder haben erkannt, dass durch eine derartige zweite Identifikationsnummer Manipulationssicherheit garantiert werden kann, ohne zusätzliche Informationen in der überwachten Vorrichtung zu speichern.

Nach einem weiteren Aspekt der Erfindung ist der zweite Datensatz ein Abfrageteil eines einheitlichen Ressourcenzeigers, wobei das zweite Übertragen den Aufruf des einheitlichen Ressourcenzeigers umfasst. Ein englischer Fachbegriff für einen einheitlichen Ressourcenzeigers ist "Uniform Ressource Locator", kurz "URL". Ein englischer Fachbegriff für den Abfrageteil eines einheitlichen Ressourcenzeigers ist "Query". Ein einheitlicher Ressourcenzeiger kann insbesondere ein elektronischer Verweis sein. Ein englischer Fachbegriff für einen elektronischen Verweis ist "Hyperlink". Das zweite Übertragen kann dann durch das Aufrufen des Hyperlinks durchgeführt werden, wobei der Abfrageteil an das Ziel des Aufrufs übertragen wird. Die Erfinder haben erkannt, dass durch die Verwendung eines einheitlichen Ressourcenzeigers bestehende Kommunikationsinfrastruktur verwendet werden kann. Bei dieser Kommunikationsinfrastruktur kann es sich beispielsweise um das Internet handeln. Es ist daher nicht notwendig, eine kostenintensive eigene Kommunikationsinfrastruktur aufzubauen und/oder vorzuhalten.

Die Erfindung kann weiterhin eine überwachte Vorrichtung betreffen, ausgebildet zum Übertragen eines Datensatzes, umfassend folgende Einheiten:
- Erste Recheneinheit, ausgebildet zum Feststellen einer Konfigurationsänderung an der überwachten Vorrichtung, weiterhin ausgebildet zum ersten Bestimmen eines ersten Datensatzes, wobei der erste Datensatz wenigstens eine öffentliche Geräteinformation und eine Konfigurationsinformation umfasst, und wobei die öffentliche Geräteinformation der überwachten Vorrichtung eineindeutig zuordenbar ist, weiterhin ausgebildet zum zweiten Bestimmen einer visuellen Information basierend auf dem ersten Datensatz,
- Ausgabeeinheit, ausgebildet zum optischen ersten Übertragen der visuellen Information zu einer Eingabeeinheit einer mobilen Überwachungseinheit.

Eine solche überwachte Vorrichtung kann insbesondere dazu ausgebildet sein, die zuvor beschriebenen erfindungsgemäßen Verfahren und ihre Aspekte auszuführen. Die überwachte Vorrichtung ist dazu ausgebildet, diese Verfahren und ihre Aspekte auszuführen, indem die erste Recheneinheit und die Ausgabeeinheit dazu ausgebildet sind, die entsprechenden Verfahrensschritte auszuführen.

Die Erfindung betrifft weiterhin ein Datenübertragungssystem, ausgebildet zum Übertragen eines Datensatzes, umfassend folgende Vorrichtungen:
a) Überwachte Vorrichtung, umfassend folgende Einheiten:
   - Erste Recheneinheit, ausgebildet zum Feststellen einer Konfigurationsänderung an der überwachten Vorrichtung, weiterhin ausgebildet zum ersten Bestimmen eines ersten Datensatzes, wobei der erste Datensatz wenigstens eine öffentliche Geräteinformation und eine Konfigurationsinformation umfasst, und wobei die öffentliche Geräteinformation der überwachten Vorrichtung eineindeutig zuordenbar ist, weiterhin ausgebildet zum zweiten Bestimmen einer visuellen Information basierend auf dem ersten Datensatz,
   - Ausgabeeinheit, ausgebildet zum optischen ersten Übertragen der visuellen Information zu einer Eingabeeinheit einer mobilen Überwachungseinheit,
b) Mobile Überwachungseinheit, umfassend folgende Einheiten:
   - Eingabeeinheit, ausgebildet zum optischen ersten Empfangen der visuellen Information von der Ausgabeeinheit der überwachten Vorrichtung,
   - Zweite Recheneinheit, ausgebildet zum dritten Bestimmen eines zweiten Datensatzes aus der visuellen Information mittels einer zweiten Recheneinheit der mobilen Überwachungseinheit, wobei aus dem zweiten Datensatz wenigstens die öffentliche Geräteinformation und die Konfigurationsinformation bestimmbar sind,
   - Schnittstelle, ausgebildet zum zweiten Übertragen des zweiten Datensatzes zu einer zentralen Datenerfassungseinheit.

Ein solches Datenübertragungssystem kann insbesondere dazu ausgebildet sein, die zuvor beschriebenen erfindungsgemäßen Verfahren und ihre Aspekte auszuführen. Das Datenübertragungssystem ist dazu ausgebildet, diese Verfahren und ihre Aspekte auszuführen, indem die überwachte Vorrichtung und ihre zugehörigen Einheiten sowie die mobile Überwachungseinheit und ihre zugehörigen Einheiten dazu ausgebildet sind, die entsprechenden Verfahrensschritte auszuführen.

Die Erfindung kann auch ein Computerprogrammprodukt mit einem Computerprogramm sowie ein computerlesbares Medium für eine überwachte Vorrichtung oder für ein Datenübertragungssystem betreffen. Eine weitgehend softwaremäßige Realisierung hat den Vorteil, dass auch schon bisher verwendete überwachte Vorrichtungen oder Datenübertragungssystem auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten sowie Hardwarekomponenten wie z. B. Hardwareschlüssel (Dongels etc.) zur Nutzung der Software umfassen.

Die Erfindung betrifft auch ein Computerprogrammprodukt mit einem Computerprogramm, welches direkt in einen Speicher eines Datenübertragungssystems ladbar ist, mit Programmabschnitten, am alle Schritte des Verfahrens nach einem der Ansprüche 1 bis 12 auszuführen, wenn die Programmabschnitte von dem Datenübertragungssystem ausgeführt werden.

Die Erfindung betrifft auch ein Computerlesbares Speichermedium, auf welchem von Datenübertragungssystem ausführbare Programmabschnitte gespeichert sind, um alle Schritte des Verfahrens nach einem der Ansprüche 1 bis 12 auszuführen, wenn die Programmabschnitte dem Datenübertragungssystem ausgeführt werden.

Bei einer überwachten Vorrichtung handelt es sich um eine medizintechnische Vorrichtung, insbesondere um eine bildgebende medizintechnische Vorrichtung. Eine überwachte Vorrichtung kann insbesondere mobil ausgebildet sein, so dass sie in unterschiedlichen Räumlichkeiten eingesetzt werden kann.

Als Konfiguration wird hier insbesondere der Zustand oder der Status der überwachten Vorrichtung beschrieben. Der Zustand der überwachten Vorrichtung umfasst insbesondere auch die verbauten Hardwarekomponenten oder die installierten Softwarekomponenten. Eine Konfigurationsinformation ist eine Information über die aktuelle Konfiguration der überwachten Vorrichtung oder eine Information über eine Änderung der Konfiguration der überwachten Vorrichtung. Eine Änderung der Konfiguration ist insbesondere eine Änderung der installierten Softwarekomponenten, oder eine Änderung der verbauten Hardwarekomponenten, oder eine Änderung des aktuellen Status der überwachten Vorrichtung.

Eine visuelle Information ist insbesondere eine derart in einem Medium enthaltene Information, so dass die visuelle Information über das Medium mittels elektromagnetischer Strahlung im sichtbaren Spektrum übertragen werden kann, wobei die elektromagnetische Strahlung insbesondere eine Wellenlänge zwischen 200 nm und 800 nm aufweist, insbesondere eine Wellenlänge zwischen 380 nm und 640 nm. Eine visuelle Information kann insbesondere auch leer sein. Die visuelle Information kann insbesondere auch identisch mit dem zweiten Datensatz sein. Weiterhin umfasst die visuelle Information insbesondere zumindest indirekt die öffentliche Geräteinformation und die Konfigurationsinformation, insbesondere kann die visuelle Information die öffentliche Geräteinformation und die Konfigurationsinformation umfassen.

Die Helligkeit einer Farbe kann insbesondere die photometrische Helligkeit der Farbe bezeichnen. Bei einer ersten Farbe und bei einer zweiten Farbe kann es sich insbesondere jeweils um einen Grauton handeln. Ein Grauton ist beispielsweise im sogenannten Rot-Grün-Blau-Modell (kurz "RGB-Modell") dadurch gegeben, dass alle drei Farbkomponenten den gleichen Wert haben. Die erste Farbe kann insbesondere dadurch im RGB-Modell gegeben sein, dass alle Farbkomponenten einen Wert von unter 50% des Maximalwerts, insbesondere von unter 10% des Maximalwerts, insbesondere von unter 1% des Maximalwerts aufweisen, die erste Farbe kann daher insbesondere auch Schwarz sein. Die zweite Farbe kann insbesondere dadurch im RGB-Modell gegeben sein, dass alle Farbkomponenten einen Wert von über 50% des Maximalwerts, insbesondere von über 90% des Maximalwerts, insbesondere von über 99% des Maximalwerts aufweisen, die zweite Farbe kann daher insbesondere auch Weiß sein. Hierbei bezeichnet der Maximalwert den maximalen Wert einer Farbkomponente.

Eine Einwegstreufunktion bildet derart Eingabewerte auf Ausgabewerte ab, dass es höchstens durch großen Rechenaufwand möglich ist, zu einem gegebenen Ausgabewert einen zugehörigen Eingabewert zu finden, der durch die Einwegstreufunktion auf den gegebenen Ausgabewert abgebildet wird. Hierdurch kann eine Einwegstreufunktion dazu verwendet werden, Daten fälschungssicher zu übertragen.

Eine Einwegstreufunktion kann insbesondere eine schwach kollisionsresistente Einwegstreufunktion sein. Für eine schwach kollisionsresistente Einwegstreufunktion ist es höchstens durch großen Rechenaufwand möglich, zu einem gegebenen ersten Eingabewert einen vom ersten Eingabewert verschiedenen zweiten Eingabewert zu finden, so dass der gegebene erste Eingabewert und der zweite Eingabewert auf den gleichen Ausgabewert abgebildet werden. Eine Einwegstreufunktion kann insbesondere eine stark kollisionsresistente Einwegstreufunktion sein. Für eine stark kollisionsresistente Einwegstreufunktion ist es höchstens durch großen Rechenaufwand möglich, einen ersten Eingabewert und einen vom ersten Eingabewert verschiedenen zweiten Eingabewert zu finden, so dass der gegebene erste Eingabewert und der zweite Eingabewert auf den gleichen Ausgabewert abgebildet werden. Hierbei ist das Finden eines Wertes insbesondere höchstens durch großen Rechenaufwand möglich, wenn kein anzuwendender Algorithmus zur Bestimmen des Wertes existiert und der Wert nur durch systematisches Probieren von möglichen Lösungen gefunden werden kann (ein englischer Fachbegriff ist "brute force"), insbesondere wenn das systematische Probieren von einem einzelnen aus dem Stand der Technik bekannten Prozessor nicht in weniger als einem Jahr durchgeführt werden kann.

Ein einheitlicher Ressourcenbezeichner (ein englischer Fachbegriff ist "Uniform Resource Identifier", kurz "URI") wird im Standard RFC 3986 definiert und besteht aus einer Zeichenfolge zur Bezeichnung von Ressourcen, insbesondere von Webseiten, Dateien und Webservices. Neben der Angabe des Fundorts einer Ressource kann ein URI auch einen Abfrageteil enthalten (ein englischer Fachbegriff ist "query"). Ein einheitlicher Ressourcenzeiger (ein englischer Fachbegrif ist "Uniform Resource Locator", kurz "URL") ist eine URI, die zusätzliche Angaben zum Auffinden der Ressource durch Angabe des primären Zugangsmechanismus aufweist. Ein Synonym für "URI" ist "Hyperlink" oder "Link".

Es zeigen:
Fig. 1 ein Flussdiagramm eines Ausführungsbeispiels des Verfahrens,
Fig. 2 eine überwachte Vorrichtung zusammen mit einer mobilen Überwachungseinheit sowie einer zentralen Datenerfassungseinheit,
Fig. 3 eine Ausgabeeinheit einer überwachten Vorrichtung sowie eine Eingabeeinheit einer mobilen Überwachungseinheit,
Fig. 4 einen ersten Datensatz mit einer zugehörigen visuellen Information,
Fig. 5 einen QR-Code,
Fig. 6 einen Strichcode.

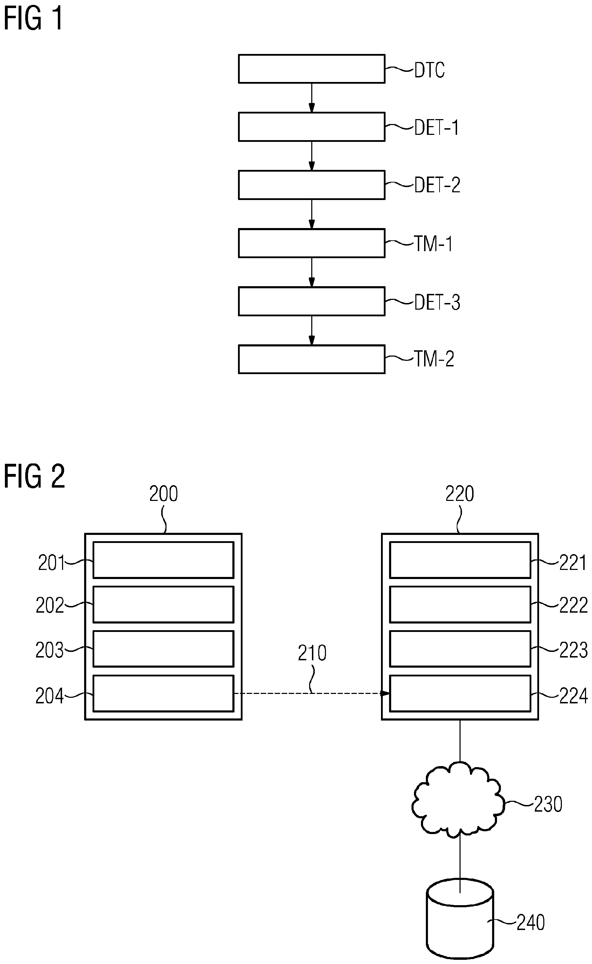

Fig. 1 zeigt ein Flussdiagramm eines ersten, eines zweiten und eines dritten Ausführungsbeispiels des erfindungsgemäßen Verfahrens.

Beim ersten Ausführungsbeispiel handelt es sich bei der überwachten Vorrichtung 200 um eine mobile medizintechnische Vorrichtung, insbesondere um ein mobiles Ultraschallgerät, welches nicht mit dem Internet verbunden ist. Auf diesem Ultraschallgerät soll vom Anwender eine neue Softwareversion installiert werden, die dem Anwender mittels eines Speichermediums zur Verfügung gestellt werden, der Erfolg der Softwareinstallation soll dann an eine Zentrale gemeldet werden. Es ist hier auch denkbar, dass einzelne Softwarekomponenten (beispielsweise Apps oder spezielle Protokolle) installiert und/oder entfernt werden.

Beim zweiten Ausführungsbeispiel handelt es sich bei der überwachten Vorrichtung 200 um ein mobiles Röntgengerät, dessen Röntgenröhre durch den Anwender ausgetauscht wird. In diesem Fall wird beispielsweise eine Identifikationsnummer der neu eingebauten Röntgenröhre an den Hersteller kommuniziert, damit dieser eine eindeutige Zuordnung zwischen den Röntgengeräten und den verbauten Röntgenröhren herstellen kann.

Beim dritten Ausführungsbeispiel wechselt das mobile Röntgengerät aufgrund eines Fehlers vom Betriebszustand in einen Fehlerzustand übergeht oder umgekehrt, diese Information soll an den Hersteller übermittelt werden.

Alternativ kann es sich bei der überwachten Vorrichtung 200 um andere Vorrichtungen handeln, beispielsweise um einen Computertomographen, einen Magnetresonanztomographen, einen Positronen-Emissions-Tomographen, ein Labordiagnostikgerät oder einen Roboter handeln.

Der erste Schritt in den dargestellten Ausführungsbeispielen ist das Feststellen DTC einer Konfigurationsänderung einer überwachten Vorrichtung 200 mittels einer ersten Recheneinheit 202 der überwachten Vorrichtung 202.

Im ersten Ausführungsbeispiel erfolgt das Feststellen DTC durch ein Signal, welches bei der Softwareinstallation durch ein Installationsprogramm an die Recheneinheit 202 geschickt wird. Alternativ kann das Feststellen DTC auch durch ein Computerprogramm durchgeführt werden, welches dauerhaft auf der Recheneinheit 202 der überwachten Vorrichtung 200 ausgeführt wird und Änderungen an der Versionsnummer der auf der überwachten Vorrichtung ausgeführten Software feststellen kann, weiterhin kann dieses Computerprogram das Hinzufügen oder das Entfernen von Softwarekomponenten feststellen.

Beim zweiten und beim dritten Ausführungsbeispiel wird das Feststellen DTC auch durch ein Computerprogramm durchgeführt, welches dauerhaft auf der Recheneinheit 202 der überwachten Vorrichtung 200 ausgeführt wird und die aktuelle Hardwarekonfiguration und/oder den aktuellen Zustand der überwachten Vorrichtung 200 überwacht. Dieses Computerprogramm kann im ersten Ausführungsbeispiel insbesondere eine Soll-Liste der in der überwachten Vorrichtung 200 verbauten Hardwarebestandteile mit der zugehörigen Identifikationsnummer speichern, und diese Liste mit den tatsächlichen Identifikationsnummern der verbauten Hardwareteile vergleichen. Weiterhin kann dieses Computerprogramm Änderungen an der Versionsnummer der auf der überwachten Vorrichtung 200 ausgeführten Software feststellen, weiterhin kann dieses Computerprogram das Hinzufügen oder das Entfernen von Softwarekomponenten feststellen. Weiterhin kann dieses Computerprogramm den Zustand der überwachten Vorrichtung 200, insbesondere Fehlermeldungen, überwachen.

Der nächste Schritt in den dargestellten Ausführungsbeispielen ist das erste Bestimmen DET-1 eines ersten Datensatzes 400 mittels der ersten Recheneinheit 202 der überwachten Vorrichtung 200, wobei der erste Datensatz 400 wenigstens eine öffentliche Geräteinformation 401 und eine Konfigurationsinformation 402 umfasst, und wobei die öffentliche Geräteinformation 401 der überwachten Vorrichtung 200 eineindeutig zuordenbar ist. In den dargestellten Ausführungsbeispielen ist die öffentliche Geräteinformation 401 die Seriennummer der überwachten Vorrichtung 200. Die Seriennummer der überwachten Vorrichtung 200 ist der überwachten Vorrichtung 200 derart eineindeutig zugeordnet, dass einer überwachten Vorrichtung 200 genau eine Seriennummer zugeordnet ist, und dass eine Seriennummer nur genau einer überwachten Vorrichtung 200 zugeordnet ist.

Im dargestellten ersten Ausführungsbeispiel ist die Konfigurationsinformation 402 die Versionsnummer der Softwareversion, die bei der Konfigurationsänderung auf der überwachten Vorrichtung installiert wird. Wird alternativ eine Softwarekomponente hinzugefügt oder entfernt, handelt es sich bei der Konfigurationsinformation 402 um eine Identifikationsnummer der hinzugefügten oder der entfernten Softwarekomponente, beispielsweise um einen Hashwert der ausführbaren Installations- oder Deinstallationsroutine. Im zweiten Ausführungsbeispiel ist die Konfigurationsinformation 402 eine Identifikationsnummer der neuen Röntgenröhre, insbesondere eine Seriennummer der neuen Röntgenröhre. Diese Konfigurationsinformation 402 kann beispielsweise auf einer auslesbaren Speichereinheit in der Röntgenröhre gespeichert sein. Im dritten Ausführungsbeispiel umfasst die Konfigurationsinformation 402 den vorherigen Zustand der überwachten Vorrichtung 200, den neuen Zustand der überwachten Vorrichtung 200 und eine Zeitinformation, wobei die Zeitinformation den Zeitpunkt der Zustandsänderungen angibt.

Der erste Datensatz 400 umfasst im ersten Ausführungsbeispiel weiterhin das Ergebnis 403 der Anwendung einer Einwegstreufunktion auf eine Kombination der öffentlichen Geräteinformation 401, der Konfigurationsinformation 402 sowie einer geheimen Geräteinformation 404. Die Einwegstreufunktion wird hierbei auch als Hashfunktion bezeichnet, das Ergebnis 403 der Anwendung der Hashfunktion wird auch als Hashwert 403 bezeichnet. Hierbei ist die geheime Geräteinformation 404 nur auf der überwachten Vorrichtung 200 und in der zentralen Datenerfassungseinheit 240 gespeichert. Die geheime Geräteinformation 404 ist hingegen nicht durch die mobile Überwachungseinheit 220 bestimmbar. Dadurch ist es für den Anwender der mobilen Überwachungseinheit 220 nicht möglich, aus der öffentlichen Geräteinformation 401 sowie der Konfigurationsinformation 402 den Hashwert 403 zu berechnen.

Die Hashfunktion bildet in diesem ersten Ausführungsbeispiel genau einen Eingabewert auf einen zugehörigen Hashwert 403 ab. Um die Hashfunktion auf die öffentliche Geräteinformation 401, die Konfigurationsinformation 402 und die geheime Geräteinformation 404 anzuwenden, wird im dargestellten Ausführungsbeispiel die Hashfunktion auf eine Verkettung einer der öffentlichen Geräteinformation 401 entsprechenden Zeichenkette, einer der Konfigurationsinformation 402 entsprechenden Zeichenkette und einer der geheimen Geräteinformation 404 entsprechenden Zeichenkette angewendet. Eine Zeichenkette entspricht insbesondere einer Information, wenn der Informationsgehalt der Zeichenkette und der Information identisch sind. Eine Verkettung von Zeichenketten kann insbesondere durch das Erstellen einer neuen Zeichenkette erfolgen, so dass die verketten Zeichenketten Teilzeichenketten der neuen Zeichenkette sind. Handelt es sich bei der öffentlichen Geräteinformation 401, bei der Konfigurationsinformation 402 oder der geheimen Geräteinformation 404 um Zahlen, ist eine zugehörige Zeichenkette insbesondere die Ziffernfolge, welche die Zahl im Dezimalsystem, im Hexadezimalsystem oder im Binärsystem darstellt. Alternativ zur Verkettung von Zeichenketten kann insbesondere aus mehreren Information genau ein Eingabewert für die Hashfunktion erstellt werden, indem die mehreren Informationen durch eine arithmetische Operation oder durch eine Zeichenkettenoperation in eine neue Information umgewandelt werden.

Im dargestellten ersten Ausführungsbeispiel wird als Hashfunktion die SHA-1 (englisches Akronym für "Secure Hash Algorithm 1", eine deutsche Übersetzung ist "Sicherer Hashalgorithmus 1" oder "Sicherer Streuwertalgorithmus 1") Hashfunktion verwendet, die in der RFC 3174 spezifiziert ist. Es ist auch möglich, andere Hashfunktionen zu verwenden. Bekannte Hashfunktionen sind MD5 (englisches Akronym für "Message-Digest Algorithm 5", eine deutsche Übersetzung ist "Nachrichtenverarbeitungsalgorithmus 5"), SHA-2 (insbesondere SHA-224, SHA-256, SHA-384, SHA-512, SHA-512/224, SHA-512/256) und SHA-3 (insbesondere SHA3-224, SHA3-256, SHA3-384, SHA3-512, SHAKE128 und HAKE256). Weiterhin sind die Hashfunktionen BLAKE, Grøstl, JH und Skein bekannt. Es ist natürlich auch möglich, andere als die hier aufgeführten Hashfunktionen zu verwenden. Dabei ist die Wahl der konkreten Hashfunktion unerheblich für den Ablauf des erfindungsgemäßen Verfahrens.

Der nächste Schritt der dargestellten Ausführungsbeispiele ist das zweite Bestimmen DET-2 einer visuellen Information 420 basierend auf dem ersten Datensatz 400 mittels der ersten Recheneinheit 202 der überwachten Vorrichtung 200. Bei der visuellen Information 420 handelt es sich im dargestellten ersten Ausführungsbeispiel um einen Link, der den ersten Datensatz 400 enthält. Dabei ist der erste Datensatz 400 der Abfrageteil des Links. Die visuelle Information 420 in Form des Links ist in einem QR-Code 500 codiert. Alternativ können auch andere eindimensionale oder zweidimensionale Barcodes verwendet werden. Als zweidimensionale Barcodes sind beispielsweise Matrix-Barcodes wie eine Aztec Code (nach dem Standard ISO/IEC 24778) oder eine Data Matrix (nach dem Standard ISO/IEC 16022), sowie gestapelte Barcodes wie ein PDF417-Code (nach dem Standard ISO/IEC 15438) bekannt.

Im zweiten und im dritten Ausführungsbeispiel handelt es sich bei der visuellen Information 420 Daten in einem für die mobile Überwachungseinheit 220 auslesbarem Format, die in einem Strichcode 600 kodiert sind. Als auslesbare Formate sind insbesondere XML und JSON bekannt. Als Strichcodes 600 sind EAN-Codes (nach dem Standard ISO/IEC 15420) oder "Code 128" (nach dem Standard ISO/IEC 15417) bekannt. Ein Strichcode 600 ist eine Unterform eines eindimensionalen Barcodes.

Es ist auch möglich, andere eindimensionale oder zweidimensionale Barcodes zu verwenden. Insbesondere ist es auch möglich, nicht-standardisierte Barcodes zu verwenden. Die Art des verwendeten Barcodes ist dabei nicht von der Art der überwachten Vorrichtung 200 oder von den Inhalten des ersten Datensatzes 400 oder der visuellen Information 420 abhängig, sondern lediglich von den technischen Möglichkeiten der Ausgabeeinheit 204 und der Eingabeeinheit 224. Weiterhin ist der Ablauf des erfindungsgemäßen Verfahrens unabhängig von der Art des verwendeten Barcodes.

Alternativ zu den dargestellten Ausführungsbeispielen ist es ebenfalls möglich, dass die visuelle Information 420 eine Zeichenkette ist, die auf einer Ausgabeeinheit 204 der überwachten Vorrichtung 200 angezeigt wird. Der Anwender kann diese Zeichenkette mittels einer Eingabeeinheit 224 der mobilen Überwachungseinheit erfassen, insbesondere mittels einer Tastatur oder einer Bildschirmtastatur.

Weiterhin ist es alternativ zu den dargestellten Ausführungsbeispielen möglich, dass abhängig von der Konfigurationsinformation 402 die visuelle Information 420 leer ist, und das keine visuelle Information 420 auf der Ausgabeeinheit 204 der überwachten Vorrichtung 200 angezeigt wird. Dies kann insbesondere der Fall sein, wenn die Konfigurationsänderung in Form einer Installation, eines Entfernens oder eines Änderns an der Hard- und/oder Software an der überwachten Vorrichtung 200 nicht erfolgreich war. Wenn keine visuelle Information 420 angezeigt wird, so kann kein zweiter Datensatz an die zentrale Datenerfassungseinheit 240 weitergegeben werden, und in der zentralen Datenerfassungseinheit 240 wird der überwachten Vorrichtung 200 kein Erfolg der Konfigurationsänderung zugeordnet. Üblicherweise wird in einer solchen Alternative in der zentralen Datenerfassungseinheit 240 der überwachten Vorrichtung 200 standardmäßig ein Misserfolg der Konfigurationsänderung gespeichert, wobei der Speicherwert nur bei einer Erfolgsmeldung geändert wird. Die weiteren Schritte des Verfahrens werden dann in diesem Ausführungsbeispiel lediglich bei einem Erfolg der Konfigurationsänderung ausgeführt.

Der nächste Schritt der dargestellten Ausführungsbeispiele ist das optische erste Übertragen TM-1 der visuellen Information 420 von einer Ausgabeeinheit 204 der überwachten Vorrichtung 200 zu einer Eingabeeinheit 224 einer mobilen Überwachungseinheit 220. Das optische erste Übertragen TM-1 erfolgt hier dadurch, dass der QR-Code 500 oder der Strichcode 600 auf der Ausgabeeinheit 204 der überwachten Vorrichtung angezeigt wird, wobei der QR-Code 500 oder der Strichcode 600 die visuelle Information 420 kodieren. Bei der Ausgabeeinheit 204 handelt es sich hierbei um einen Bildschirm. Alternativ kann es sich bei der Ausgabeeinheit 204 auch um einen Drucker handeln, welcher den QR-Code 500 oder den Strichcode 600 auf ein Papier ausdruckt. Bei der Eingabeeinheit 224 der mobilen Überwachungseinheit handelt es sich hier um eine Kamera. Diese Kamera ist dazu ausgebildet, ein Bild eines QR-Codes 500 oder des Strichcodes 600 aufzunehmen. Das optische erste Übertragen TM-1 erfolgt hierbei derart, dass die Kamera der mobilen Überwachungseinheit ein Bild der Ausgabeeinheit 204, hier insbesondere des Bildschirms, der überwachten Vorrichtung 200 macht. Alternativ kann es sich bei der Eingabeeinheit 224 auch um eine Scan-Einheit handeln. Weiterhin kann es sich bei der Eingabeeinheit 224 auch um eine Tastatur handeln, mit der ein Anwender die auf der Ausgabeeinheit 204 dargestellte Information in die mobile Überwachungseinheit eingeben kann.

Als nächster Schritt der dargestellten Ausführungsbeispiele erfolgt das dritte Bestimmen DET-3 eines zweiten Datensatzes aus der visuellen Information 420 mittels einer zweiten Recheneinheit 222 der mobilen Überwachungseinheit 220, wobei aus dem zweiten Datensatz wenigstens die öffentliche Geräteinformation 401 und die Konfigurationsinformation 402 bestimmbar sind. In den dargestellten Ausführungsbeispielen wird durch die zweite Recheneinheit 222 der QR-Code 500 oder der Strichcode 600 erkannt, die visuelle Information 420 extrahiert und in einen zweiten Datensatz umgewandelt, wobei der zweite Datensatz ein von der zentralen Datenerfassungseinheit 220 lesbares Format aufweist. Die visuelle Information 420 kann insbesondere identisch mit dem zweiten Datensatz sein.

Der letzte Schritt der dargestellten Ausführungsbeispiele ist das zweite Übertragen TM-2 des zweiten Datensatzes von einer Schnittstelle 221 der mobilen Überwachungseinheit 220 zu einer zentralen Datenerfassungseinheit 240. Im dargestellten ersten Ausführungsbeispiel erfolgt das zweite Übertragen TM-2 derart, dass der im QR-Code 500 enthaltene Link aufgerufen wird. Der Adressteil des Links verweist hierbei auf die zentrale Datenerfassungseinheit 240, durch Aufruf des Links wird die im Abfrageteil enthaltene Information über das Internet 230 an die zentrale Datenerfassungseinheit 240 weitergegeben. Die zentrale Datenerfassungseinheit kann dann aufgrund der von ebenfalls in ihr gespeicherten geheimen Geräteinformation 404 den zweiten Datensatz verifizieren. Anschließend kann sie die Konfigurationsinformation 402 in dem zur überwachten Vorrichtung gehörigen Datensatz speichern, indem sie die öffentliche Geräteinformation der überwachten Vorrichtung 200 zuordnet. Im zweiten und im dritten Ausführungsbeispiel kommuniziert die Applikation auf der mobilen Überwachungseinheit 220 über ein vorher definiertes Protokoll mit der zentralen Überwachungseinheit, beispielsweise indem eine E-Mail mit einer vorher definierten Formatierung verschickt wird. Bei einer zentralen Datenerfassungseinheit 240 kann es sich insbesondere um eine Datenbank oder einen Datenbankserver handeln, alternativ kann es sich bei der zentralen Datenerfassungseinheit 240 auch um einen Webserver oder einen E-Mailserver handeln.

Fig. 2 zeigt eine überwachte Vorrichtung 200 zusammen mit einer mobilen Überwachungseinheit 220 und einer zentralen Datenerfassungseinheit 240. Die überwachte Vorrichtung umfasst hierbei eine Schnittstelle 201, eine erste Recheneinheit 202, eine erste Speichereinheit 203 sowie eine Ausgabeeinheit 204. Die mobile Überwachungseinheit 220 umfasst eine Schnittstelle 221, eine zweite Recheneinheit 222, eine zweite Speichereinheit 223 sowie eine Eingabeeinheit 224. Die Ausgabeeinheit 204 der überwachten Vorrichtung 200 ist mit der Eingabeeinheit 224 der mobilen Überwachungseinheit 220 über einen optischen Kanal 210 verbunden. Weiterhin ist die mobile Überwachungseinheit 220 über ein Netzwerk 230 mit der zentralen Datenerfassungseinheit 240 verbunden. Die überwachte Vorrichtung 200 kann insbesondere einen Computer, einen Mikrocontroller oder einen integrierten Schaltkreis umfassen, welche die Schnittstelle 201, die erste Recheneinheit 202, die erste Speichereinheit 203 und/oder die erste Ausgabeeinheit 204 umfassen. Bei einer mobilen Überwachungseinheit 220 kann es sich ebenfalls insbesondere um einen Computer, einen Mikrocontroller oder um einen integrierten Schaltkreis handeln. Weiterhin kann eine mobile Überwachungseinheit 220 insbesondere ein Mobiltelefon, insbesondere ein Smartphone sein. Bei einer Schnittstelle 201, 221 kann es sich um eine Hardware- oder Softwareschnittstelle handeln (beispielsweise PCI-Bus, USB oder Firewire). Eine erste Recheneinheit 202 und eine zweite Recheneinheit 222 kann Hardwareelemente oder Softwareelemente aufweisen, beispielsweise einen Mikroprozessor oder ein sogenanntes FPGA (englisches Akronym für "Field Programmable Gate Array"). Eine erste Speichereinheit 203 und eine zweite Speichereinheit 223 kann als nicht dauerhafter Arbeitsspeicher (Random Access Memory, kurz RAM) oder als dauerhafter Massenspeicher (Festplatte, USB-Stick, SD-Karte, Solid-Date-Disc) realisiert sein. Eine Ausgabeeinheit 204 kann insbesondere ein Bildschirm sein, es kann sich alternativ aber auch um einen Drucker handeln. Bei einer Eingabeeinheit 224 handelt es sich insbesondere um eine Kamera. Es kann sich aber auch um eine Tastatur, um eine Maus oder um einen Scanner. Bei einem Netzwerk 230 kann es sich um ein Intranet oder insbesondere um das Internet handeln. Eine zentrale Datenerfassungseinheit zu 240 kann insbesondere durch eine Datenbank realisiert sein, die zentrale Datenerfassungseinheit 240 kann weiterhin noch eine Recheneinheit zur Datenverwaltung umfassen.

Die Eingabeeinheit 224 der mobilen Überwachungseinheit 220 ist dazu ausgebildet, die von der Ausgabeeinheit 204 der überwachten Vorrichtung 200 angezeigt visuelle Information 420 zu erfassen. Die visuelle Information 420 wird dabei über ein optisches Medium 210 übertragen, wobei das optische Medium hier die Umgebungsluft ist. Hierbei kann die visuelle Information 420 insbesondere mittels eines Bildes dargestellt oder kodiert werden, insbesondere mittels eines QR-Codes 500 oder eines Strichcodes 600. Handelt es sich beispielsweise wie im gezeigten Ausführungsbeispiel bei der Ausgabeeinheit 204 um einen Bildschirm, so kann die Eingabeeinheit 224 als Kamera ausgebildet sein, die einen auf dem Bildschirm dargestellten QR-Code 500 aufnehmen kann. Handelt es sich bei der Ausgabeeinheit 204 um einen Drucker, der Papier mit der visuellen Information 420 bedruckt, kann es sich bei der Eingabeeinheit 224 ebenfalls um eine Kamera oder um einen Scanner handeln.

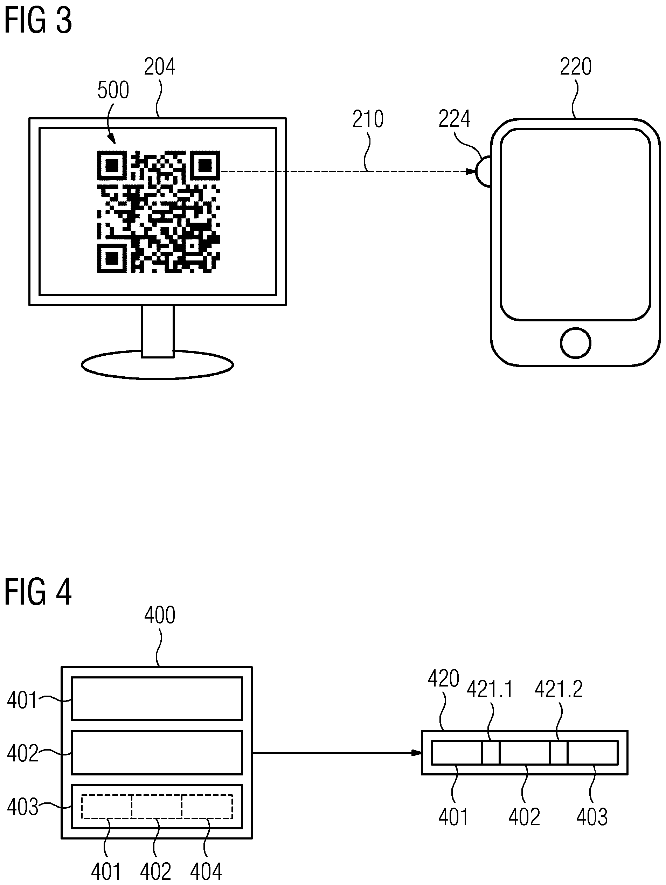

Fig. 3 zeigt eine Ausgabeeinheit 204 einer überwachten Vorrichtung 200 und eine Eingabeeinheit 224 einer mobilen Überwachungseinheit 220 im ersten Ausführungsbeispiel. Die Ausgabeeinheit 204 ist hier ein Bildschirm oder Monitor der mobilen Ultraschalleinheit, der dazu ausgebildet ist, einen QR-Code 500 anzuzeigen. Die mobile Überwachungseinheit 220 ist in diesem Ausführungsbeispiel als Mobiltelefon ausgeführt, insbesondere als Smartphone, ihre Eingabeeinheit 224 als Kamera, insbesondere als Fotokamera, die dazu ausgebildet ist, einen QR-Code 500 zu erfassen. Das optische Übertragungsmedium 210 ist hierbei durch die Umgebungsluft ausgebildet. Das Mobiltelefon kann über ein Mobilfunknetz oder über ein drahtloses Netzwerk (beispielsweise WLAN) mit dem Netzwerk 230 (hier das Internet) und damit mit der zentralen Datenerfassungseinheit 240 kommunizieren.

Fig. 4 zeigt den ersten Datensatz 400 sowie eine zugehörige visuelle Information 420 im ersten Ausführungsbeispiel. Der erste Datensatz 400 umfasst eine öffentliche Geräteinformation 401, eine Konfigurationsinformation 402 sowie einen Hashwert 403. Der Hashwert 403 wurde durch Anwendung einer Hashfunktion bestimmt, wobei die Hashfunktion auf eine Kombination der öffentlichen Geräteinformation 401, der Konfigurationsinformation 402 sowie einer geheimen Geräteinformation 404 angewendet wurde. Im dargestellten Ausführungsbeispiel handelt es sich bei der öffentlichen Geräteinformation 401 um eine Seriennummer, bei der Konfigurationsinformation 402 handelt es sich um eine Softwareversionsnummer, bei der geheimen Geräteinformation 404 handelt es sich um eine geheime Seriennummer. Die Kombination der öffentlichen Geräteinformation 401, der Konfigurationsinformation 402 sowie der geheimen Geräteinformation 404 erfolgt derart, dass die Informationen in eine Zeichenkette umgewandelt werden, und die Zeichenketten verknüpft werden. Die Hashfunktion wird dann auf die verknüpfte Zeichenkette angewendet. Die visuelle Information 420 umfasst ebenfalls die öffentliche Geräteinformation 401, die Konfigurationsinformation 402 sowie den Hashwert 403, sowie Trennzeichen 421.1, 421.2. Diese Informationen werden als Zeichenketten hintereinander verknüpft. Die dadurch entstehende Zeichenkette kann optional als QR-Code 500 oder als Strichcode 600 kodiert werden, es sind aber auch andere Kodierungen möglich.

Im dargestellten Ausführungsbeispiel kann beispielsweise ein Hyperlink der folgenden Form verwendet werden: http://example.com/verify.php?s=123&c=2&h=654

Hierbei bezeichnet "http" das in der URL verwendete Schema, "example.com" den Host der URL, der hier der Adresse der zentralen Datenerfassungseinheit 240 entspricht, "verify.php" den Pfad der URL und "s=123&c=2&h=654" den Abfrageteil der URL. Im Abfrageteil werden durch "s=123" die öffentliche Geräteinformation 401, durch "c=2" die Konfigurationsinformation 402 und durch h="654" der Hashwert 404 aufgelistet, die einzelnen Informationen werden durch das Et-Zeichen "&" als Trennzeichen 421.1, 421.2 getrennt. Durch Aufruf des Links wird der Abfrageteil an die zentrale Datenerfassungseinheit 240 geschickt.

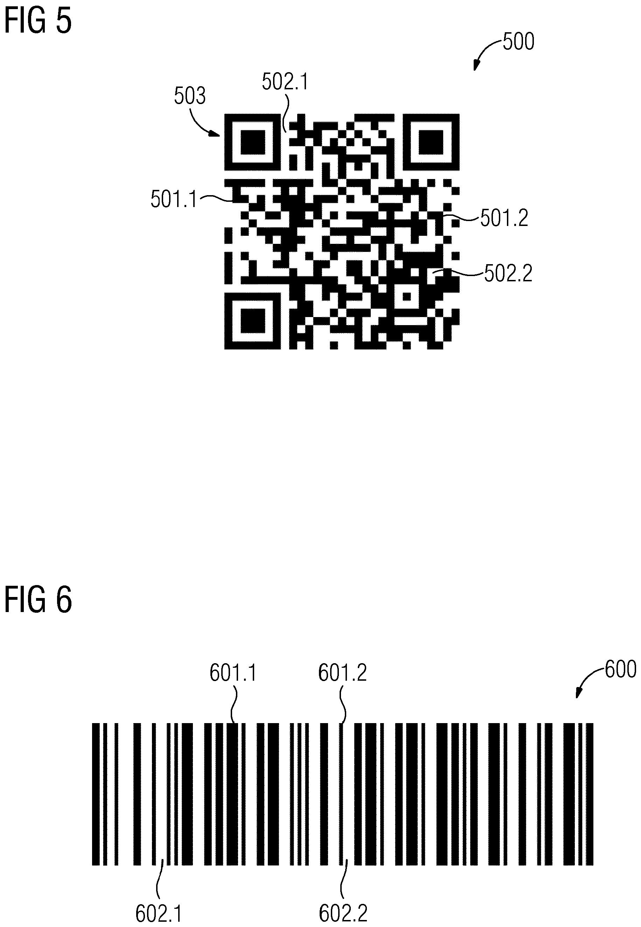

Fig. 5 zeigt einen QR-Code 500. Der QR-Code 500 besteht aus ersten Bereichen 501.1, 501.2 in schwarzer Farbe sowie zweiten Bereichen 502.1, 502.2 in weißer Farbe. Dabei sind die ersten Bereiche 501.1, 501.2 eine Ansammlung von schwarzen Pixeln, die zweiten Bereiche 502.1, 502.2 sind eine Ansammlung von weißen Pixeln. Die Pixel des QR-Codes 500 sind in einer Gitterstruktur angeordnet. Weiterhin umfasst der QR-Code 500 Positionsmarken 503 in Form einer geometrischen Anordnung von weißen und schwarzen Pixeln, ein QR-Code 500 kann noch weitere Marken oder Muster enthalten, welche eine Erfassung des QR-Codes 500 und/oder eine Dekodierung des QR-Codes 500 erleichtern. Die Information des QR-Codes 500 ist in der geometrischen Form der ersten Bereiche 501.1, 501.2 und der zweiten Bereiche 502.1, 502.2 enthalten. Dabei ist die geometrische Form der ersten Bereiche 501.1, 501.2 und der zweiten Bereiche 502.1, 502.2 in diesem Ausführungsbeispiel zusammenhängende quadratische Pixel in einem Gitter gegeben.

Ein QR-Code 500 kann redundante Informationen enthalten. Diese redundanten Informationen können insbesondere dazu genutzt werden, Fehler in der Darstellung oder in der Erfassung von QR-Codes 500 auszugleichen. Dabei sind verschiedene Redundanzstufen möglich, insbesondere mehr als 7% redundanter Inhalt, insbesondere mehr als 15% redundanter Inhalt, insbesondere mehr als 25% redundanter Inhalt, insbesondere mehr als 30% redundanter Inhalt. Hierbei heißt ein Inhalt des QR-Codes 500 redundant, wenn die im QR-Code 500 enthaltene visuelle Information 420 auch ohne diesen Inhalt bestimmt werden kann. Zur Implementierung der Fehlerkorrektur ist insbesondere die Reed-Solomon-Codierung bekannt.

Fig. 6 zeigt einen Strichcode 600. Der Strichcode besteht aus schwarzen Strichen 601.1, 601.2 sowie weißen Strichen 602.1 und 602.2. Die im Strichcode 600 encodierte Information ist in den unterschiedlichen Dicken der Striche 601.1, 601.2, 602.1 und 602.2 enthalten. Der Strichcode 600 kann ebenfalls redundante Informationen enthalten, so dass eine Fehlerkorrektur beim Erfassen des Strichcodes 600 möglich ist. Insbesondere ist es bekannt, Prüfzeichen zu verwenden.

## Patentansprüche

1. Verfahren zum Übertragen eines Datensatzes, umfassend die folgenden Verfahrensschritte:
- Feststellen (DTC) einer Konfigurationsänderung an einer überwachten Vorrichtung (200) mittels einer ersten Recheneinheit (202) der überwachten Vorrichtung (200),
wobei die überwachte Vorrichtung (200) eine medizintechnische Vorrichtung ist,
- Erstes Bestimmen (DET-1) eines ersten Datensatzes (400) mittels der ersten Recheneinheit (202) der überwachten Vorrichtung (200),
wobei der erste Datensatz (400) wenigstens eine öffentliche Geräteinformation (401) und eine Konfigurationsinformation (402) umfasst,
und wobei die öffentliche Geräteinformation (401) der überwachten Vorrichtung (200) eineindeutig zuordenbar ist,
- Zweites Bestimmen (DET-2) einer visuellen Information (420) basierend auf dem ersten Datensatz (400) mittels der ersten Recheneinheit (202) der überwachten Vorrichtung (200),
- Optisches erstes Übertragen (TM-1) der visuellen Information (420) von einer Ausgabeeinheit (204) der überwachten Vorrichtung (200) zu einer Eingabeeinheit (224) einer mobilen Überwachungseinheit (220),
- Drittes Bestimmen (DET-3) eines zweiten Datensatzes aus der visuellen Information (420) mittels einer zweiten Recheneinheit (222) der mobilen Überwachungseinheit (220),
wobei aus dem zweiten Datensatz wenigstens die öffentliche Geräteinformation (401) und die Konfigurationsinformation (402) bestimmbar sind,
- Zweites Übertragen (TM-2) des zweiten Datensatzes von einer Schnittstelle (221) der mobilen Überwachungseinheit (220) zu einer zentralen Datenerfassungseinheit (240).

2. Verfahren nach Anspruch 1, wobei die Konfigurationsinformation (402) wenigstens eine Information über den Erfolg der Konfigurationsänderung an der überwachten Vorrichtung (200) umfasst.

3. Verfahren nach Anspruch 1 oder 2, wobei die visuelle Information (420) in einem Bild enthalten ist, und wobei beim optischen ersten Übertragen (TM-1) das Bild auf der Ausgabeeinheit (204) der überwachten Vorrichtung (200) dargestellt wird und das Bild von der Eingabeeinheit (224) der mobilen Überwachungseinheit (220) erfasst wird.

4. Verfahren nach Anspruch 3, wobei das Bild wenigstens einen ersten Bildbereich (501.1, 501.2, 601.1, 601.2) und einen zweiten Bildbereich umfasst (502.1, 502.2, 602.1, 602.2) wobei der wenigstens eine erste Bildbereich (501.1, 501.2, 601.1, 601.2) eine erste Farbe aufweist und der wenigstens eine zweite Bildbereich (502.1, 502.2, 602.1, 602.2) eine zweite Farbe aufweist, und wobei sich die erste Farbe und die zweite Farbe zumindest in ihrer Helligkeit und/oder ihrem Farbton unterscheiden.

5. Verfahren nach Anspruch 4, wobei die visuelle Information (420) in der geometrischen Anordnung des wenigstens einen ersten Bildbereiches (501.1, 501.2, 601.1, 601.2) und des wenigstens einen zweiten Bildbereiches (502.1, 502.2, 602.1, 602.2) des Bildes enthalten ist.

6. Verfahren nach Anspruch 5, wobei das Bild einen eindimensionalen Barcode (600) oder einen zweidimensionalen Barcode (500) umfasst.

7. Verfahren nach einem der vorherigen Ansprüche, wobei die öffentliche Geräteinformation (401) eine erste Identifikationsnummer der überwachten Vorrichtung (200) umfasst.

8. Verfahren nach einem der vorherigen Ansprüche,
wobei die Konfigurationsänderung das Installieren und/oder das Entfernen einer Hardware- und/oder Softwarekomponente umfasst,
und wobei die Konfigurationsinformation (402) eine zweite Identifikationsnummer der Hardware- und/oder Softwarekomponente umfasst.

9. Verfahren nach einem der vorherigen Ansprüche, wobei die visuelle Information (420) derart ausgebildet ist, dass eine Veränderung der Konfigurationsinformation (402) und der öffentlichen Geräteinformation (401) mittels der mobilen Überwachungseinheit (220) und/oder mittels der zentralen Datenerfassungseinheit (240) feststellbar ist.

10. Verfahren nach Anspruch 9, wobei die visuelle Information (420) wenigstens das Ergebnis (403) der Anwendung einer Einwegstreuwertfunktion auf den ersten Datensatz (400) umfasst.

11. Verfahren nach Anspruch 10,
wobei der erste Datensatz (400) weiterhin eine geheime Geräteinformation (404) umfasst,
wobei die geheime Geräteinformation (404) der überwachten Vorrichtung (200) eineindeutig zuordenbar ist,
und wobei die geheime Geräteinformation (404) nicht aus der visuellen Information (420) bestimmbar ist.

12. Verfahren nach einem der vorherigen Ansprüche, wobei der zweite Datensatz ein Abfrageteil eines einheitlichen Ressourcenzeigers ist, und wobei das zweite Übertragen (TM-2) den Aufruf des einheitlichen Ressourcenzeigers umfasst.

13. Datenübertragungssystem, ausgebildet zum Übertragen eines Datensatzes, umfassend folgende Vorrichtungen:
a) Überwachte Vorrichtung (200), wobei die überwachte Vorrichtung (200) eine medizintechnische Vorrichtung ist, umfassend folgende Einheiten:
- Erste Recheneinheit (202), ausgebildet zum Feststellen (DTC) einer Konfigurationsänderung an der überwachten Vorrichtung (200),
weiterhin ausgebildet zum ersten Bestimmen (DET-1) eines ersten Datensatzes (400), wobei der erste Datensatz (401) wenigstens eine öffentliche Geräteinformation (401) und eine Konfigurationsinformation (402) umfasst, und wobei die öffentliche Geräteinformation (401) der überwachten Vorrichtung (200) eineindeutig zuordenbar ist,
weiterhin ausgebildet zum zweiten Bestimmen (DET-2) einer visuellen Information (420) basierend auf dem ersten Datensatz (400),
- Ausgabeeinheit (204), ausgebildet zum optischen ersten Übertragen (TM-1) der visuellen Information (420) zu einer Eingabeeinheit (224) einer mobilen Überwachungseinheit (220),
b) Mobile Überwachungseinheit (220), umfassend folgende Einheiten:
- Eingabeeinheit (224), ausgebildet zum optischen ersten Empfangen (TM-1) der visuellen Information (420) von der Ausgabeeinheit (204) der überwachten Vorrichtung (200),
- Zweite Recheneinheit (222), ausgebildet zum dritten Bestimmen (DET-3) eines zweiten Datensatzes aus der visuellen Information (420),
wobei aus dem zweiten Datensatz wenigstens die öffentliche Geräteinformation (401) und die Konfigurationsinformation (402) bestimmbar sind,
- Schnittstelle (221), ausgebildet zum zweiten Übertragen (TM-2) des zweiten Datensatzes zu einer zentralen Datenerfassungseinheit (240).

14. Datenübertragungssystem nach Anspruch 13, weiterhin ausgebildet ein Verfahren nach einem der Ansprüche 2 bis 12 auszuführen.

15. Computerprogrammprodukt mit einem Computerprogramm, welches direkt in einen Speicher (203) eines Datenüberwachungssystems ladbar ist, mit Programmabschnitten, um das Feststellen (DTC), das erste Bestimmen (DET-1), das zweite Bestimmen (DET-2) und das optische erste Übertragen (TM-1) des Verfahrens nach einem der Ansprüche 1 bis 12 auszuführen, wenn die Programmabschnitte von dem Datenüberwachungssystem ausgeführt werden.

16. Computerlesbares Speichermedium, auf welchem von einem Datenüberwachungssystem ausführbare Programmabschnitte gespeichert sind, um das Feststellen (DTC), das erste Bestimmen (DET-1), das zweite Bestimmen (DET-2) und das optische erste Übertragen (TM-1) des Verfahrens nach einem der Ansprüche 1 bis 12 auszuführen, wenn die Programmabschnitte von dem Datenüberwachungssystem ausgeführt werden.

## Claims

1. Method for transmitting a data record, comprising the following method steps:
- detection (DTC) of a configuration change at a monitored device (200) by means of a first computer unit (202) of the monitored device (200),
wherein the monitored device (200) is a medical device,
- first determination (DET-1) of a first data record (400) by means of the first computer unit (202) of the monitored device (200),
wherein the first data record (400) comprises at least one item of public device information (401) and an item of configuration information (402),
and wherein the item of public device information (401) is uniquely assignable to the monitored device (200),
- second determination (DET-2) of an item of visual information (420) on the basis of the first data record (400) by means of the first computer unit (202) of the monitored device (200),
- optical first transmission (TM-1) of the item of visual information (420) from an output unit (204) of the monitored device (200) to an input unit (224) of a mobile monitoring unit (220),
- third determination (DET-3) of a second data record from the item of visual information (420) by means of a second computer unit (222) of the mobile monitoring unit (220),
wherein from the second data record, at least the item of public device information (401) and the item of configuration information (402) are determinable,
- second transmission (TM-2) of the second data record from an interface (221) of the mobile monitoring unit (220) to a central data acquisition unit (240).

2. Method according to claim 1, wherein the item of configuration information (402) comprises at least one item of information concerning the success of the configuration change at the monitored device (200).

3. Method according to claim 1 or 2, wherein the item of visual information (420) is contained in an image, and wherein on the optical first transmission (TM-1), the image is displayed on the output unit (204) of the monitored device (200) and the image is acquired by the input unit (224) of the mobile monitoring unit (220).

4. Method according to claim 3, wherein the image comprises at least one first image region (501.1, 501.2, 601.1, 601.2) and a second image region (502.1, 502.2, 602.1, 602.2), wherein the at least one first image region (501.1, 501.2, 601.1, 601.2) has a first colour and the at least one second image region (502.1, 502.2, 602.1, 602.2) has a second colour and wherein the first colour and the second colour differ at least in their brightness and/or their colour tone.

5. Method according to claim 4, wherein the item of visual information (420) is contained in the geometric arrangement of the at least one first image region (501.1, 501.2, 601.1, 601.2) and the at least one second image region (502.1, 502.2, 602.1, 602.2) of the image.

6. Method according to claim 5, wherein the image comprises a one-dimensional barcode (600) or a two-dimensional barcode (500).

7. Method according to one of the preceding claims, wherein the item of public device information (401) comprises a first identification number of the monitored device (200).

8. Method according to one of the preceding claims,
wherein the configuration change comprises the installation and/or the removal of a hardware and/or software component, and wherein the item of configuration information (402) comprises a second identification number of the hardware and/or software component.

9. Method according to one of the preceding claims, wherein the item of visual information (420) is configured such that a change of the item of configuration information (402) and of the item of public device information (401) is detectable by means of the mobile monitoring unit (220) and/or by means of the central data acquisition unit (240).

10. Method according to claim 9, wherein the item of visual information (420) comprises at least the result (403) of the use of a one-way hash function on the first data record (400).

11. Method according to claim 10,
wherein the first data record (400) further comprises an item of secret device information (404),
wherein the item of secret device information (404) is assignable one-to-one to the monitored device (200),
and wherein the item of secret device information (404) is not determinable from the item of visual information (420).

12. Method according to one of the preceding claims, wherein the second data record is a query part of a uniform resource locator, wherein the second transmission (TM-2) comprises the invoking of the uniform resource locator.

13. Data transmission system, configured to transmit a data record, comprising the following devices:
a) a monitored device (200), wherein the monitored device (200) is a medical device, comprising the following units:
- a first computer unit (202), configured for detecting (DTC) a configuration change at the monitored device (200),
further configured for first determination (DET-1) of a first data record (400), wherein the first data record (400) comprises at least one item of public device information (401) and an item of configuration information (402), and wherein the item of public device information (401) is assignable one-to-one to the monitored device (200),
further configured for second determination (DET-2) of an item of visual information (420) based on the first data record (400),
- an output unit (204), configured for optical first transmission (TM-1) of the item of visual information (420) to an input unit (224) of a mobile monitoring unit (220),
b) a mobile monitoring unit (220), comprising the following units:
- an input unit (224), configured for the optical first reception (TM-1) of the item of visual information (420) from the output unit (204) of the monitored device (200),
- a second computer unit (222), configured for the third determination (DET-3) of a second data record from the item of visual information (420),
wherein from the second data record, at least the item of public device information (401) and the item of configuration information (402) are determinable,
- an interface (221), configured for second transmission (TM-2) of the second data record to a central data acquisition unit (240).

14. Data transmission system according to claim 13, which is further configured to carry out a method according to one of claims 2 to 12.

15. Computer program product having a computer program which is directly loadable into a memory store (203) of a data monitoring system, having program portions in order to carry out the detection (DTC), the first determination (DET-1), the second determination (DET-2) and the optical first transmission (TM-1) of the method according to one of claims 1 to 12 when the program portions are executed by the data monitoring system.

16. Computer-readable storage medium on which program portions executable by a data monitoring system are stored in order to carry out the detection (DTC), the first determination (DET-1), the second determination (DET-2) and the optical first transmission (TM-1) of the method according to one of claims 1 to 12 when the program portions are executed by the data monitoring system.

## Revendications

1. Procédé de transmission d'un ensemble de données comportant les étapes de procédé suivantes :
- une constatation (DTC) d'une modification de configuration à un dispositif (200) surveillé au moyen d'un premier processeur (202) du dispositif (200) surveillé,
dans lequel le dispositif (200) surveillé est un dispositif de la technique médicale,
- une première détermination (DET-1) d'un premier ensemble (400) de données au moyen du premier processeur (202) du dispositif (200) surveillé,
dans lequel le premier ensemble (400) de données comporte au moins une information (401) d'appareil public et une information (402) de configuration,
et dans lequel l'information (401) d'appareil public du dispositif (200) surveillé peut être affecté de manière bijective au dispositif (200) surveillé,
- une deuxième détermination (DET-2) d'une information (420) visuelle basée sur le premier ensemble (400) de données au moyen du premier processeur (202) du dispositif (200) surveillé,
- une première transmission (TM-1) optique de l'information (420) visuelle à partir d'une unité (204) de sortie du dispositif (200) surveillé vers une unité (224) d'entrée d'une unité (220) de surveillance mobile,
- une troisième détermination (DET-3) d'un deuxième ensemble de données à partir de l'information (420) visuelle au moyen d'un deuxième processeur (222) de l'unité (220) de surveillance mobile, dans lequel, à partir du deuxième ensemble de données, au moins l'information (401) d'appareil public et l'information (402) de configuration peuvent être déterminés,
- une deuxième transmission (TM-2) du deuxième ensemble de données à partir d'une interface (221) de l'unité (220) de surveillance mobile vers une unité (240) centrale de détection de données.

2. Procédé suivant la revendication 1, dans lequel l'information (402) de configuration comporte au moins une information concernant le succès de la modification de configuration au dispositif (200) surveillé.

3. Procédé suivant la revendication 1 ou 2, dans lequel l'information (420) visuelle est contenue dans une image et dans lequel, lors de la première transmission (TM-1) optique, l'image est affichée sur l'unité (204) de sortie du dispositif (200) surveillé et l'image est détectée par l'unité (224) d'entrée de l'unité (220) de surveillance mobile.

4. Procédé suivant la revendication 3, dans lequel l'image comporte au moins une première région d'image (501.1, 501.2, 601.1, 601.2) et une deuxième région d'image (502.1, 502.2, 602.1, 602.2), dans lequel la au moins une première région d'image (501.1, 501.2, 601.1, 601.2) a une première couleur et la au moins une deuxième région d'image (502.1, 502.2, 602.1, 602.2) a une deuxième couleur, et dans lequel les première et deuxième couleurs diffèrent au moins dans leurs clartés et/ou leur teintes de couleurs.

5. Procédé suivant la revendication 4, dans lequel l'information (420) visuelle est contenue dans l'agencement géométrique de la au moins une première région d'images (501.1, 501.2, 601.1, 601.2) et de la au moins une deuxième région d'images (502.1, 502.2, 602.1, 602.2) de l'image.

6. Procédé suivant la revendication 5, dans lequel l'image comporte un code-barres (600) à une dimension ou un code-barres (500) à deux dimensions.

7. Procédé suivant l'une des revendications précédentes, dans lequel l'information (401) d'appareil public comporte un premier numéro d'identification du dispositif (200) surveillé.

8. Procédé suivant l'une des revendications précédentes, dans lequel la modification de configuration comporte l'installation et/ou le retrait d'un composant hardware et/ou d'un composant software, et dans lequel l'information (402) de configuration comporte un deuxième numéro d'identification du composant hardware et/ou software.

9. Procédé suivant l'une des revendications précédentes, dans lequel l'information (420) visuelle est configurée de sorte qu'une modification de l'information (402) de configuration et de l'information (401) d'appareil public peut être constatée au moyen de l'unité (220) de surveillance mobile et/ou au moyen de l'unité (240) centrale de détection de données.

10. Procédé suivant la revendication 9, dans lequel l'information (420) visuelle inclut au moins le résultat (403) de l'utilisation d'une fonction de hachage à une voie sur le premier ensemble (400) de données.

11. Procédé suivant la revendication 10, dans lequel le premier ensemble (400) de données comporte en outre une information (404) d'appareil secret, dans lequel l'information (404) d'appareil secret peut être associée de manière bijective au dispositif (200) surveillé, et dans lequel l'information (404) d'appareil secret ne peut pas être déterminée à partir de l'information (420) visuelle.

12. Procédé suivant l'une des revendications précédentes, dans lequel le deuxième ensemble de données est une partie de question d'un dispositif de localisation de ressources unitaire, et dans lequel la deuxième transmission (TM-2) inclut le fait d'invoquer le dispositif de localisation de ressources unitaire.

13. Système de transmission de données, réalisé pour transmettre un ensemble de données, comportant les dispositifs suivants :
a) un dispositif (200) surveillé, le dispositif surveillé étant un dispositif de technique de la médecine, comportant les unités suivantes :
- un premier processeur (202), configuré pour constater (DTC) une modification de configuration au dispositif (200) surveillé,
configuré en outre pour une première détermination (DET-1) d'un premier ensemble (400) de données, le premier ensemble (401) de données comportant au moins une information (401) d'appareil public et une information (402) de configuration et l'information (401) d'appareil public pouvant être associée de manière bijective au dispositif (200) surveillé,
configuré en outre pour une deuxième détermination (DET-2) d'une information (420) visuelle basée sur le premier ensemble (400) de données,
- une unité (204) de sortie, configurée pour une première transmission (TM-1) optique de l'information (420) visuelle à une unité (224) d'entrée d'une unité (220) de surveillance mobile,
b) une unité (220) de surveillance mobile comportant les unités suivantes :
- une unité (224) d'entrée configurée pour une première réception (TM-1) optique de l'information (420) visuelle en provenance de l'unité (204) de sortie du dispositif (200) surveillé,
- un deuxième processeur (222) configuré pour une troisième détermination (DET-3) d'un deuxième ensemble de données à partir de l'information (420) visuelle,
dans lequel l'information (401) d'appareil public et l'information (402) de configuration peuvent être déterminées à partir du deuxième ensemble de données,
- une interface (221), configurée pour une deuxième transmission (TM-2) du deuxième ensemble de données à une unité (240) centrale de détection de données.

14. Système de transmission de données suivant la revendication 13, configuré en outre pour mettre en œuvre un procédé suivant l'une des revendications 2 à 12.

15. Produit de programme d'ordinateur comportant un programme d'ordinateur, lequel peut être chargé directement dans une mémoire (203) d'un système de surveillance de données, comportant des parties de programme pour mettre en œuvre la constatation (DTC), la première détermination (DET-1), la deuxième détermination (DET-2) et la première transmission (TM-1) optique du procédé suivant l'une des revendications 1 à 12, lorsque les parties de programme sont exécutées par le système de surveillance de données.

16. Moyen de stockage pouvant être lu par ordinateur, sur lequel des parties de programme pouvant être mises en œuvre par un système de surveillance de données sont mémorisées, afin de mettre en œuvre la constatation (DTC), la première détermination (DET-1), la deuxième détermination (DET-2) et la première transmission (TM-1) optique du procédé suivant l'une des revendications 1 à 12, lorsque les parties de programme sont exécutées par le système de surveillance de données.
